# EUROPÄISCHE PATENTSCHRIFT

(11) **EP 0 402 433 B1**
(45) Veröffentlichungstag und Bekanntmachung des Hinweises auf die Patenterteilung: **16.06.1993**
(21) Anmeldenummer: 89911770.9
(22) Anmeldetag: 23.10.1989
(51) Int. Cl.: A61B 17/22, G10K 15/04, A61B 17/36

(54) **VORRICHTUNG ZUM ZERTRÜMMERN EINES FESTEN VON EINER FLÜSSIGKEIT UMGEBENEN KÖRPERS**
DEVICE FOR BREAKING UP A SOLID BODY SURROUNDED BY A FLUID
DISPOSITIF POUR DETRUIRE UN CORPS SOLIDE ENTOURE D'UN FLUIDE

(30) Priorität: 25.10.1988 DE 3836336; 25.10.1988 DE 3836337; 27.10.1988 DE 3836525
(43) Veröffentlichungstag der Anmeldung: 19.12.1990
(73) Patentinhaber: STORZ MEDICAL AG, CH-8280 Kreuzlingen (CH)
(72) Erfinder: MEESSEN, Stephan, D-5300 Bonn 1 (DE)
(74) Vertreter: Münich, Wilhelm, Dr.
(86) Internationale Anmeldenummer: DE8900677
(87) Internationale Veröffentlichungsnummer: WO9004358

(56) Entgegenhaltungen:
- EP-A- 0 144 764
- EP-A- 0 268 019
- Biomedizinische Technik, volume 30, Nr. 7-8, 1985, (Berlin DE),H.Schmidt-Kloiber et al: "Laserinduced shock-wave lithotripsy (LISL)",pages 173-181

## Beschreibung

### Technisches Gebiet

Die Erfindung bezieht sich auf eine Vorrichtung zur Zertrümmerung eines von einem Fluid umgebenen festen Körpers, wie beispielsweise von Konkrementen im harnableitenden System, im Gallen- bzw. Gallengangsbereich oder im Speichelgang lebender Körper gemäß dem Oberbegriff des Anspruchs 1.

### Stand der Technik

Laserinduzierte Stoßwellen werden beispielsweise zur Zertrümmerung von Steinen bzw. Konkrementen in Organen oder Gefäßen, wie dem harnableitenden System, im Gallen- bzw. Gallengangsbereich oder im Speichelgang lebender Körper oder zur Beseitigung von Verkalkungen oder Verstopfungen in Gefäßen eingesetzt. Laserinduzierte Stoßwellen werden durch ein mit Überschallgeschwindigkeit expandierendes Plasma erzeugt, das wiederum dadurch erzeugt wird, daß der Laserstrahl auf ein geeignetes Medium, das ein Festkörper, eine Flüssigkeit oder ein Gas sein kann, einwirkt. Die auch als Durchbruch bezeichnete Durchbruchskatalyse, die die eigentliche Plasma-Reaktion auslöst, erfordert eine hohe momentane Aktivationsenergie, die über der zur Aufrechterhaltung der Plasma-Reaktion benötigten Energie liegt. Hierzu wird auf die Artikel "Laser-induced High-Pressure Shock Waves in Water" in Applied Physics Letter, Vol.10, 1967, S.46 folgende, oder "Laserinduced Shock-Wave Lithotripsy" in Biomedizinische Technik, Bd. 30, S.173 bis 181, verwiesen.

Insbesondere dann, wenn als Laser ein Laser verwendet wird, der Licht im roten Teil des sichtbaren Spektrums oder im nahen Infrarot emittiert, kann es vorkommen, daß ein Laserpuls aufgrund schlechter Fokussierung, einem ungeeigneten Medium im Fokusbereich etc. auch bei großer Pulsenergie keinen optischen Durchbruch auslöst.

Deshalb ist in der EP-A-0 268 019 vorgeschlagen worden, im Bereich des Fokus des Lasers als "Katalysator" für den optischen Durchbruch beispielsweise einen Platin-Steg anzuordnen. Mit einer derartigen Ausgestaltung wird zwar die Wahrscheinlichkeit, daß ein Laserpuls einen optischen Durchbruch auslöst, vergrößert, die Lebensdauer der Vorrichtung wird jedoch herabgesetzt, da das im Bereich des Fokuspunktes angeordnete Material, also beispielsweise der Platin-Steg, hohen Belastungen ausgesetzt ist.

Weiterhin ist in EP-A-0 304 689 vorgeschlagen worden, zur Erhöhung der Auslösewahrscheinlichkeit des optischen Durchbruchs ein geeignetes Spülmedium, wie beispielsweise eine Eisen-3-Lösung zu verwenden. Viele dieser Spülmedien und insbesondere die vorstehend genannte Eisen-3-Lösung haben jedoch den Nachteil, daß sie unter das Arzneimittelgesetz fallen und somit für die in-vivo Applikation derzeit nicht einsetzbar sind.

Unabhängig davon, ob ein "Katalysator" für den laserinduzierten Durchbruch verwendet wird, besteht bei den bekannten Vorrichtungen zur Zertrümmerung eines von einem Fluid umgebenen festen Körpers noch folgendes Problem:

Die mechanische und die thermische Energie, in die beim laserinduzierten Durchbruch die optische Energie umgesetzt wird, wirkt auf den eingesetzten Lichtleiter zurück, so daß dieser nach einer mehr oder weniger großen Betriebsdauer derart beschädigt wird, daß er nicht mehr fokussiert und damit keine Stoßwellen mehr erzeugt werden können. Da die Zertrümmerung beispielsweise eines Steines im menschlichen Körper häufig erst nach einer mehrminütigen Applikation von laserinduzierten Stoßwellen, erfahrungsgemäß etwa zwischen 300 und 6000 Laserpulsen eintritt, ist die Wahrscheinlichkeit, daß der Lichtleiter während einer Operation durch Verschleiß ausfällt, sehr groß. Dabei stellt sich als weiteres Problem, daß es häufig für den behandelnden Arzt nicht möglich ist, den Funktionsausfall der Vorrichtung zu erkennen, so daß er zunächst noch die Behandlung fortsetzt und in der Regel erst dann, wenn sich ein Erfolg stellt, die Behandlung abbricht.

### Darstellung der Erfindung

Der Erfindung liegt die Aufgabe zugrunde, eine Vorrichtung zur Zertrümmerung eines von einem Fluid umgebenen festen Körpers mittels laserinduzierten Stoßwellen derart weiterzubilden, daß die Stoßwellen sicher ausgelöst werden, ohne daß die Vorrichtung einem zu großen Verschleiß unterliegt.

Diese Aufgabe wird durch die in den Ansprüchen 1 und 12 angegebene erfindungsgemäße Vorrichtung gelöst. Weiterbildungen der Erfindung sind Gegenstand der Unteransprüche.

Erfindungsgemäß emittiert die Lasereinrichtung einen zweiten Laserstrahl, dessen Wellenlänge kürzer als die des ersten Laserstrahls ist, und die zwischen etwa 170 nm und 550 nm liegt. Der zweite Laserstrahl ist ebenfalls auf das absorbierende Medium gerichtet ist, und ionisiert das Medium zum Teil.

Dabei geht die Erfindung von der Erkenntnis aus, daß freie Elektronen für das Auslösen des laserinduzierten Durchbruchs katalytisch wirken; die für den Durchbruch benötigte Aktivierungsenergie wird dabei um so niedriger, je mehr freie Elektronen vorhanden sind. Erfindungsgemäß wird deshalb ein zweiter Laserstrahl verwendet, der ebenfalls auf das absorbierende Medium gerichtet ist, und dessen Wellenlänge kürzer als die des ersten Laserstrahls ist und insbesondere zwischen etwa 170 nm und 550 nm liegt. Dieser Laserstrahl erzeugt durch Ionisation des absorbierenden Mediums die für die Herabsetzung der Durchbruchschwelle nötigen Elektronen, so daß der erste Laserstrahl den laserinduzierten Durchbruch sicher auslösen kann.

Durch diese erfindungsgemäße Ausbildung ist es nicht nur bei einem zusätzlich in dem Bereich der Laserstrahlung eingebrachten Element (Anspruch 4), wie beispielsweise ein Wolfram-Steg, sondern auch gerade dann, wenn das absorbierende Medium der zu zertrümmernde feste Körper (Anspruch 2) oder das den zu zertrümmernden festen Körper umgebende Fluid (Anspruch 3) ist, möglich, mit hoher Sicherheit einen laserinduzierten Durchbruch auszulösen, der eine energiereiche Stoßwelle zur Folge hat.

Damit wird es insbesondere möglich, mit physiologisch verträglichen und zugelassenen Materialien, wie beispielsweise Wasser, physiologischen Kochsalzlösungen, einer Zuckerlösung, einer Emulsion oder einer Suspension den laserinduzierten Durchbruch auszulösen (Anspruch 9).

Da die erfindungsgemäße Ausbildung die Erzeugung eines laserinduzierten Durchbruchs erleichtert, und somit eine Reihe von Fluiden als absorbierende Medien in Betracht kommen, ist es insbesondere gemäß Anspruch 10 möglich, unterschiedliche Fluide mit dementsprechend unterschiedlichem Brechungsindex zu verwenden, um so den Fokuspunkt der Laserstrahlung innerhalb eines durch Gerätekonstanten vorgegebenen Bereichs variieren zu können.

Dabei kann die Variation des Brechungsindex insbesondere durch Änderung der Zusammensetzung, der Konzentration und/oder Temperatur des Fluids erfolgen (Anspruch 11).

Bei der erfindungsgemäßen laserinduzierten Stoßwellenerzeugung ist es möglich, den Laserstrahl mit kurzer Wellenlänge und den Laserstrahl mit größerer Wellenlänge gleichzeitig und mit gleicher Frequenz zu pulsen (Anspruch 6). Ebenso ist es jedoch gemäß Anspruch 7 möglich, den Laserstrahl mit kürzerer Wellenlänge und den Laserstrahl mit größerer Wellenlänge abwechselnd oder zeitlich verschoben zu pulsen.

In jedem Falle ist es bevorzugt, wenn beide Laserstrahlen durch einen einzigen Lichtleiter geleitet werden (Anspruch 14), der insbesondere gemäß Anspruch 13 Bestandteil eines an sich bekannten flexiblen oder starren Endoskops ist.

Die erfindungsgemäße Vorrichtung kann gemäß Anspruch 15 zwei Laser, beispielsweise einen sogenannten Excimerlaser zum Erzeugen des Lichts kürzerer Wellenlänge und einen Infrarot-Laser, wie einen Neodym-YAG-Laser oder einen Erbium-YAG-Laser zum Erzeugen des Lichts mit längerer Wellenlänge aufweisen.

Ferner ist es aber auch möglich, daß die erfindungsgemäße Vorrichtung einen einzigen Laser aufweist, dessen Laserlicht zum Erzeugen des Lichts kürzerer Wellenlänge in bekannter Weise Frequenz-vervielfacht ist (Anspruch 16).

Als Lichtleiter können Quarzfaser-Lichtleiter, wie monofile Quarzfasern geeigneten Durchmessers verwendet werden, die sowohl die Laserstrahlung längerer Wellenlänge als auch die Laserstrahlung kürzerer Wellenlänge leiten können.

Da bei der erfindungsgemäßen Vorrichtung keine Katalysatoren für den laserinduzierten Durchbruch beispielsweise in Form von Wolfram- oder anderen Metall-Stegen benötigt werden, weist die erfindungsgemäße Vorrichtung eine größere Lebensdauer als bekannte Vorrichtung auf. Außerdem werden keinerlei Metallpartikel im Körper freigesetzt. Aber auch bei Verwendung eines Katalysators für den optischen Durchbruch wird die Lebensdauer der Vorrichtung erhöht, da mit geringeren Pulsenergien des ersten Laserstrahls gearbeitet werden kann, da durch die durch den zweiten Laserstrahl erzeugten freien Elektronen die Wahrscheinlichkeit für das Erzeugen eines optischen Durchbruchs vergrößert wird.

Dabei ist es insbesondere durch Verlegen des Fokuspunktes für die Laserstrahlen in ausreichendem Abstand vor dem Lichtleiterkopf möglich, diesen vor thermischer und/oder mechanischer Überlastung durch die induzierten Stoßwellen zu schützen. Dieser größere Abstand Fokus/Scheitel des Lichtleiters als bei bekannten Vorrichtungen ist insbesondere deshalb möglich, da die erfindungsgemäße Ionisation den Durchbruch erleichtert, so daß nicht mit Lichtstrahlen mit einem großen Öffnungswinkel gearbeitet werden muß.

Gemäß Anspruch 196 ist ein Drucksensor vorgesehen, der die in dem Fluid erzeugte rücklaufende Stoßwelle erfaßt, und dessen Ausgangssignal eine Auswerteeinheit, die beispielsweise ein Oszillograph, eine Mikrocomputer-Schaltung oder dgl sein kann, zum Erkennen des laserinduzierten Durchbruchs erfasst. Dabei ist es bevorzugt, wenn der Drucksensor extrakorporal angeordnet ist, und ein Druckübertragungsmedium, das bspw. das Spülmedium sein kann, die von der reflektierten Stoßwelle erzeugten Druckänderungen überträgt.

Dabei wird von der Überlegung ausgegangen, daß die laserinduzierte Stoßwelle nicht nur auf den zu zertrümmernden Körper wirkt, sondern von dem den Körper umgebenden Fluid auch in der entgegengesetzten Richtung übertragen wird, so daß sie bspw. durch das Spülmedium in den Kanal der Sonde zum proximalen Ende der Sonde läuft. Damit kann bspw. durch Einbringen eines Drucksensors in das Spülmedium die Stoßwelle erfasst werden. Für den Fall, daß bei einem Laserpuls keine Stoßwelle erfasst wird, ist davon auszugehen, daß kein laserinduzierter Durchbruch stattgefunden hat. Die Ursache hierfür kann bspw. sein, daß die Lichtaustrittsfläche des Lichtleiters verschliessen ist.

Weiterhin wird auch die auf den harten Gegenstand auftreffende Stoßwelle an diesem reflektiert und läuft u.a. durch das Spülmedium zum proximalen Ende der Sonde. Wenn sich nun vor dem Lichtleiterkopf kein "harter" Gegenstand befindet, sondern ausschließlich Gewebe und Flüssigkeit, so wird lediglich die durch den Laser induzierte Stoßwelle zurücklaufen. Damit kann durch Erfassen der Druckdifferenzen im Spülmedium und/oder der zeitlichen Verzögerung zwischen Druck-Maximas auf den bestand zwischen Durchbruchsort und hartem Gegenstand geschlossen werden. Ferner ist es möglich, den Zertrümmerungserfolg festzustellen, da nach dem "Verschwinden" des harten Gegenstandes auch keine Stoßwelle an diesem reflektiert wird, so daß das Verschwinden eines "zweiten Druckmaximas" nach einer Reihe von Laserpulsen ein Indiz für die Zerstörung beispielsweise eines Steines ist.

### Kurze Beschreibung der Zeichnung

Die Erfindung wird nachstehend anhand von Ausführungsbeispielen unter Bezugnahme auf die Zeichnung exemplarisch beschrieben, in der zeigen:
- Fig. 1: die Zertrümmerung eines harten Gegenstandes durch laserinduzierte Stoßwellen,
- Fig. 2: die Fokussierung der aus dem Lichtleiter austretenden Laserstrahlen,
- Fig. 3: die für die Erzeugung eines Plasmas erforderlichen Energien,
- Fig. 4: ein Ausführungsbeispiel einer erfindungsgemäßen Vorrichtung zum Erzeugen von Stoßwellen,
- Fig. 5: vergrößert Teile aus Fig. 4, und
- Fig. 6 und 7: ein weiteres Ausführungsbeispiel.

### Wege zur Ausführung der Erfindung

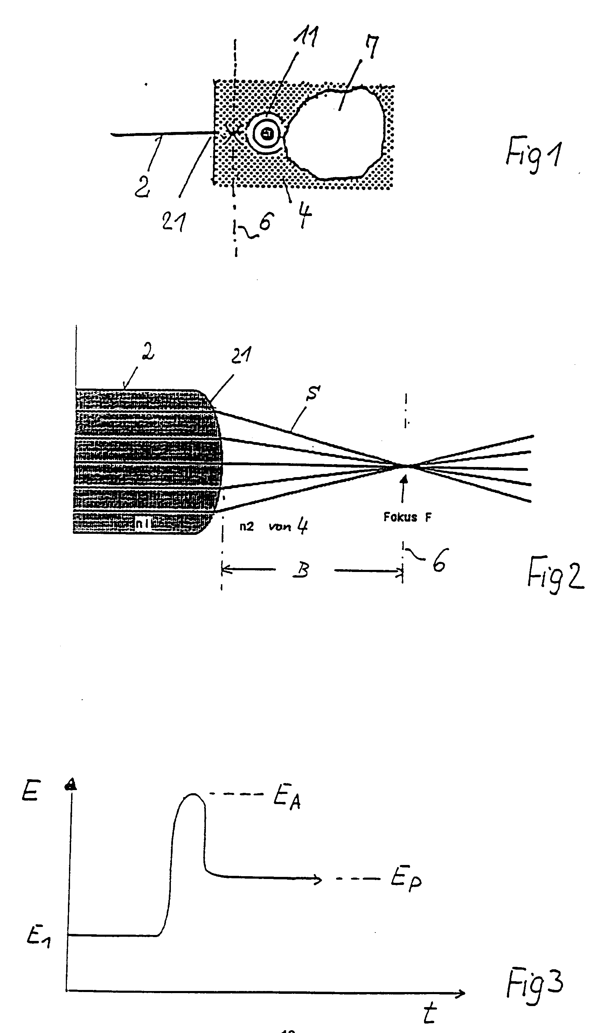

Fig. 1 zeigt schematisch die Zertrümmerung eines harten Gegenstandes 7 durch laserinduzierte Stoßwellen. Ein Lichtleiter 2 leitet einen Laserstrahl in ein den harten Körper umgebende Fluid 4. Der Laserstrahl tritt aus dem Kopf, d.h. der Fläche 21 des Lichtleiters 2 aus und löst in dem Medium 4, das sich vor dem Lichtleiterkopf 21 befindet, im Bereich 6 (i.f. auch als Durchbruchsort bezeichnet) eine Stoßwelle 11 aus, die auf den zu zertrümmernden Gegenstand 7 trifft, und auf diesen einwirkt.

Fig. 2 zeigt eine Möglichkeit für die Fokussierung der aus dem Lichtleiter 2 austretenden Laserstrahlen S. Der Lichtleiter-Austrittsfläche 21 ist konvex ausgebildet, so daß die austretenden Laserstrahlen im Fokus F fokussiert werden. Im Fokus F wird der Durchbruch ausgelöst. Vorteilhafterweise wird der Fokus F und damit der Durchbruchsort 6 soweit als möglich vor den Scheitel des Lichtleiterkopfs 21 gelegt:

Der Abstand B des Fokus F vom Scheitel des Lichtleiterkopfs 21 wird nicht nur durch die Krümmung der konvexen Austrittsfläche, sondern auch durch das Verhältnis des Brechungsindex n1 der Lichtleiterfaser 2 und des Brechungsindex n2 des umgebenden Spülmediums 4 bestimmt.

Bei vorgegebenen Brechindex n1 des Lichtleiters 2 durch die Quarzfaser kann der Abstand B des Fokus durch Vergrößerung des Brechindex n2 des Spülmediums 4 vergrößert werden und damit in einem ausreichendem bestand von dem Lichtleiterkopf 21 verlegt werden.

Die beliebige Einstellung des Fokusabstandes B kann bspw. dadurch erfolgen, daß in ein vorgegebenes Spülmedium 4 eine konzentrierte Lösung, z.B. eine Glukoselösung, eindosiert wird, und zwar so lange, bis der gewünschte Abstand B des Fokus F vom Scheitel der Fläche 21 erreicht ist.

Fig. 3 zeigt schematisch die für die Erzeugung eines Plasmas erforderlichen Energien. Für das stabile Aufrechterhalten einer Plasma-Reaktion ist eine Energie E_{P} erforderlich. Diese liegt über einer beliebigen Ausgangsenergie E1. Um jedoch den Durchbruch zu erzielen, d.h. die Plasma-Reaktion auszulösen, ist eine Aktivationsenergie E_{A} erforderlich, die größer als E_{P} ist. Erst nach dem Auslösen der plasma-Reaktion wird diese durch die geringere Energie Eₚ aufrechterhalten. Dies ist schematisch durch die auf der Abzisse in Fig. 3 aufgetragene Zeit t dargestellt.

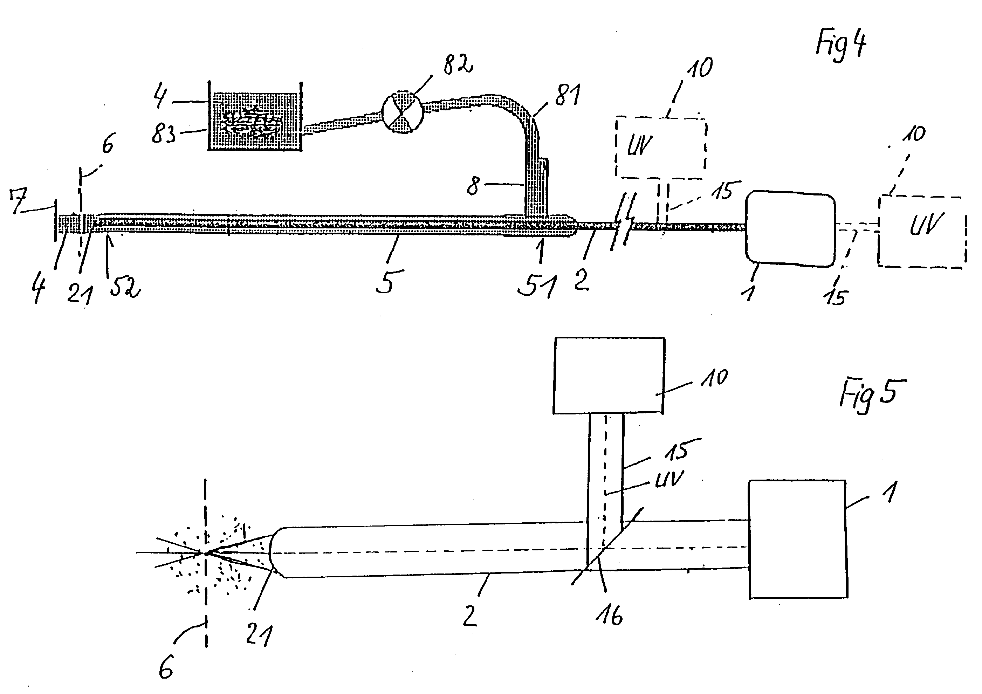

Fig. 4 zeigt ein Ausführungsbeispiel einer erfindungsgemäßen Vorrichtung zum Erzeugen von Stoßwellen für eine intrakorporale Therapie, die bspw. für die Zertrümmung von harten Gegenständen 7, wie Nierensteinen, Gallensteinen oder zur Beseitigung von Verkalkungen oder Verstopfungen in Gefäßen eingesetzt werden kann.

Das Licht eines Lasers 1, der beispielsweise Licht im IR-Bereich abgibt, wird von dem Lichtleiter 2 zu dessen distaler Lichtaustrittsfläche 21 geleitet. Dieser Laser liefert erfindungsgemäß die Energie, die die Durchbruchskatalyse am Durchbruchsort 6 bewirkt.

Bei dem gezeigten Ausführungsbeispiel für eine Vorrichtung zum Erzeugen laserinduzierter Stoßwellen ist ein zweiter Laser 10 vorgesehen, der Licht im UV-Bereich oder im blauen Bereich des sichtbaren Spektrums erzeugt. Die Laserstrahlung des UV-Lasers 10 wird über einen weiteren Lichtleiter 15 dem Lichtleiter 2 zugeführt, so daß der UV-Laserstrahl und der IR-Laserstrahl gemeinsam durch die einzige Quarzfaser 2 an derem distalen Ende 21 austreten. Es ist natürlich auch möglich, umgekehrt die IR-Laserstrahlen in den die UV-Strahlung führenden Lichtleiter einzuleiten.

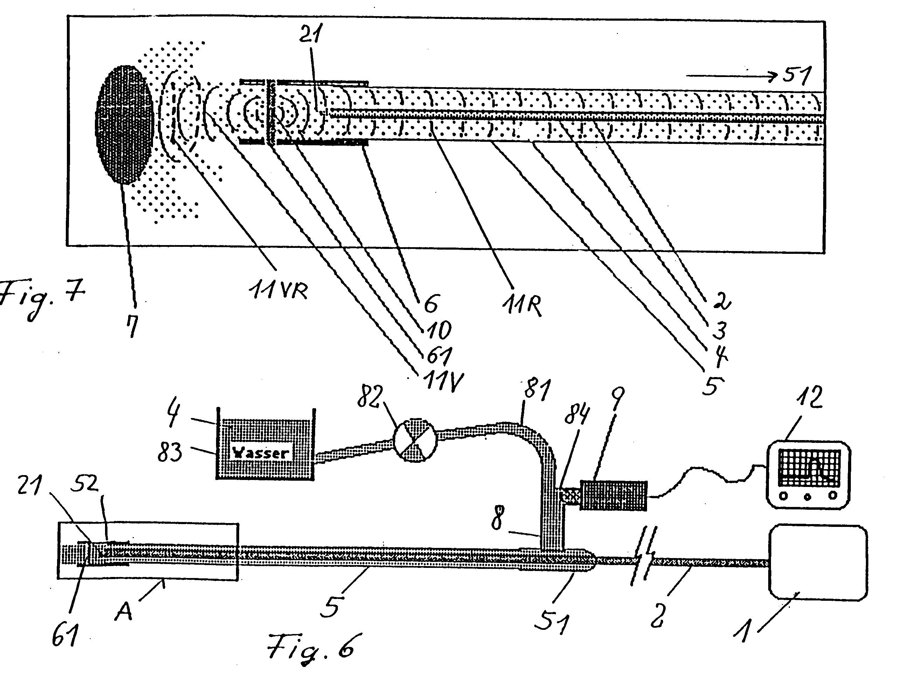

Diese Einleitung kann, wie gestrichelt dargestellt, entweder durch koaxiale Führung erfolgen oder aber auch eine mit einer Umlenkung verbundene Einleitung des einzuführenden Laserstrahles. Eine solche Umlenkung ist bspw. in Fig. 5 dargestellt: Der von dem Laser 10 erzeugte Laserstrahl wird über den Lichtleiter 15 geführt zu einem teildurchlässigen Spiegel 16 geführt, der ihn koaxial in den Lichtleiter 2, in dem der IR-Laserstrahl geführt wird, einspiegelt. Hierdurch treten beide Laserstrahlen gemeinsam am distalen Lichtleiterkopf 21 aus, und werden durch die konvexe Gestaltung des Lichtleiterkopfs 21 am Durchbruchsort 6 fokussiert.

Das Licht des Lasers 10 ionisiert das Medium 4, so daß die für die Erzeugung des Plasmas aufzubringende Akivationsenergie E_{A} herbgesetzt wird, so da die Bildung des Plasmas durch die durch den IR-Laserstrahl zugeführte Energie erleichtert und damit die Stoßwelle mit geringeren Laserpuls-Energien des Lasers 1 als beim Stand der Technik ausgelöst wird.

Der Lichtleiter 2 ist bevorzugt ein flexibler Lichtleiter, bspw. eine Quarzfaser. Die Quarzglasfaser 2 ist bspw. von einer Isolierhülle 3, bspw. aus Polytetrafluorethylen, umgeben. Ein Teil des Lichtleiters 2 ist mit einer an sich bekannten rohrförmigen Sonde 5 zum Einführen in die Körperhöhle, in der ein Konkrement etc. zertrümmert werden soll, umgeben. Die Sonde 5 ist bevorzugt flexibel, so daß sie auch gekrümmt in entsprechende Körperhöhlen eingeführt werden kann. Die Sonde, die beispielsweise ein Endoskop sein kann, ist ferner mehrlumig ausgebildet, um zumindest den Lichtleiter 2, ggf. in einem verschiebbaren Kanal und sowie wenigstens einen Kanal für das Spülmedium 4 aufzunehmen. Die rohrförmige Sonde 5 kann auch mit weiteren Kanälen, beispielsweise für eine Optik, zum Absaugen der Zertrümmerungsprodukte etc. ausgerüstet sein. Im Bereich des proximalen Endes 51 der Sonde ist ein Anschlußstutzen 8 für das Spülmedium 4, bspw. Wasser oder eine Zuckerlösung, vorgesehen, das aus einem Vorratsbehälter 83 über eine Leitung 81 mittels einer Pumpe 82 dosiert eingegeben wird. Das Spülmedium 4 tritt am distalen Ende 52 der Sonde 5 aus und umgibt den Lichtleiterkopf 21. Das Spülmedium kann selbst die plasmaerzeugende Materie bilden oder aber diese enthalten.

Die Fig. 6 und 7 zeigen ein weiteres Ausführungsbeispiel einer erfindungsgemäßen Vorrichtung, bei dem am distalem Ende 52 der Sonde 5 vor dem Lichtleiterkopf 21 mittels eines Trägers 6 ein geeignetes Element 61 angeordnet ist. Dieses Element dient als absorbierendes Medium. Im übrigen sind bei diesem Ausführungsbeispiel gleiche Teile mit gleichen Bezugszeichen versehen.

Der Wolfram-Steg 61 bildet die Durchbruchsstelle oder den Durchbruchsort 10 für die laserinduzierte Stoßwelle 2. Dies ist vergrößert in Fig. 7 dargestellt. Die von dem Lichtleiter übertragenen Laserimpulse treffen direkt auf den Wolfram-Steg 61 auf und erzeugen die Stoßwelle 11V, die in Strahlrichtung weiter auf einen harten Gegenstand, bspw. einen Stein 7 läuft. Gleichzeitig reflektiert er die am Durchbruchsort 10 erzeugte Stoßwelle, die als Stoßwelle 11R zum proximalen Ende 51 der Sonde läuft. Durch die Sonde 5 wird - wie bereits beschrieben - das Spülmedium 4, eine Flüssigkeit, eingeführt, die am distalen Sondenende 51 austritt und den Raum in der Körperhöhle und um den zu zertrümmernden Gegenstand 7 füllt. Diese Spülflüssigkeit stellt das Übertragungsmedium für die an dem Wolfram-Steg 61, d.h. dem Durchbruchsort, induzierte Stoßwelle dar. Als Spülmedium kann Wasser oder auch eine andere geeignete körperverträgliche Flüssigkeit eingesetzt werden.

Außer der vom Durchbruch 10 zurücklaufenden Stoßwelle wird auch die auf den Stein 7 auftreffende Stoßwelle 11V an diesem reflektiert und läuft gegenüber der ersten Stoßwelle 11R verzögert als zweite Stoßwelle 11VR in Richtung auf das proximale Ende. Beide Stoßwellen werden vom Spülmedium durch den Kanal in der Sonde 5 zu einem Drucksensor 9 weitergeleitet, dessen Ausgangssignal beispielsweise von einem Oszillographen 12 dargestellt wird. Der Drucksensor 9 zum Messen der in dem Spülmedium 4 vom distalen Sondenende 52 zurücklaufenden reflektierten Stoßwellen, die zum proximalen Ende der Sonde 5 wanderen, ist bei dem gezeigten Ausführungsbeispiel exemplarisch im Bereich des Anschlußstutzens 8 an einer Auslaßöffnung 84 angebracht. Es ist auch möglich, den Drucksensor 9 direkt in der Sonde 5 im Bereich des proximalen Sondenendes 51 anzuordnen, wobei die Stoßwellen möglichst frontal auf den Drucksensor auflaufen sollten.

Wenn beispielsweise durch Verschleiß des Wolfram-Steges oder der Austrittsfläche 21 des Lichtleiters 2 keine Stoßwellen mehr erzeugt werden, werden auch keine reflektierten Stoßwellen mehr vom Drucksensor 9 registriert. Damit ist eine eindeutige Überprüfung der Funktionsfähigkeit der Sonde zum Erzeugen laserinduzierter Stoßwellen auch intraoperativ möglich. Desweiteren kann mit der Vorrichtung überprüft werden, ob die Sonde mit dem distalem Ende ausreichend nahe an dem zu zertrümmernden Gegenstand 7 herangeführt ist. Erst wenn die Sonde sich dicht vor dem Stein 7 befindet, können die erzeugten Stoßwellen reflektiert werden und eine entsprechende Erfassung durch den Drucksensor 9 und Registrierung ist möglich. Dies bedeutet, daß die Sonde unter Aussenden von Laserimpulsen, ohne daß ein Endoskop eingesetzt werden muß, in eine Körperhöhle bis zu dem gewünschten Ort eingeführt werden kann. Sobald das distale Ende der Sonde sich dem zu zertrümmernden Gegenstand nähert, ändern sich die Druckanzeigen im Oszilloskop 12 und die gewünschte Plazierung kann vorgenommen werden. Nach der Zertrümmerung des Gegenstandes 7 werden keine Stoßwellen mehr an diesem reflektiert, so daß sich wiederum die Druckanzeige in dem Oszilloskop 12 ändert, so daß der Operateur weiß, daß er sein Ziel erreicht hat. Wenn der Druck ganz ausfällt, ist dies ein Anzeichen dafür, daß keine Stoßwellen mehr erzeugt werden und die Vorrichtung funktionsuntüchtig geworden ist. Das kann durch Ausfall des Kopplers oder durch Zerstörung des Lichtleiterkopfes geschehen.

Als Drucksensoren können hochempfindliche handelsüblich erhältliche Druckaufnehmer eingesetzt werden. Bei piezoelektrischen Druckaufnehmern wird das vom Druckaufnehmer abgegebene Ladungssignal üblicherweise durch einen Ladungsverstärker in eine proportionale Ausgangsspannung umgewandelt, die dann einer geeigneten Auswerteeinrichtung zugeführt wird.

Darüberhinaus ist es erfindungsgemäß auch möglich, zwei Drucksensoren gleichzeitig und in gleichem Abstand vom Durchbruchsort einzusetzen, um das Rauschen auszufiltern und die Meßgenauigkeit zu erhöhen.

So kann bspw. die erfindungsgemäß vorgenommene Erfassung der Druckänderung aufgrund der reflektierten Stoßwelle bzw. den reflektierten Stoßwellen auch intrakorporal erfolgen. Selbstverständlich kann die Druckmessung auch dann erfolgen, wenn kein Koppler bswp. in Form eines Wolfram-Steges vorgesehen ist.

Weiterhin ist es selbstverständlich nicht erforderlich, zwei Laser für die Erzeugung des Lichts in unterschiedlichen Wellenlängenbereichen vorzusehen. Vielmehr kann auch mit einem Laser, bspw. einem Neodym-YAG-Laser gearbeitet werden, bei dem ein Teil des erzeugten Laserlichts in an sich bekannter Weise zur Erzeugung von Licht kürzerer Wellenlänge Frequenz-vervielfacht wird. Eine derartige Frequenz-Vervielfachung kann selbstverständlich auch bei anderen Lasern als Neodym-YAG-Lasern erfolgen.

## Patentansprüche

1. Vorrichtung zur Zertrümmerung eines von einem Fluid (4) umgebenen festen Körpers (7), wie beispielsweise von Konkrementen im harnableitenden System, im Gallen- bzw. Gallengangsbereich oder im Speichelgang lebender Körper, mit
- einer Lasereinrichtung (1,10), die einen ersten gepulsten Laserstrahl mit einer Wellenlänge im Bereich zwischen etwa 500 nm und etwa 1400 nm emittiert,
- einem Lichtleiter (2) für den Laserstrahm, und
- einer im Anschluß an den Lichtleiter vorgesehenen Fokussiereinrichtung (21), die den Laserstrahl auf ein absorbierendes Medium derart fokussiert, daß mittels des photoakustischen Effekts bzw. des optischen Durchbruchs in dem absorbierenden Medium bzw. dem diese Medium umgebenden Fluid Stoßwellen erzeugt werden, die den festen Körper zertrümmern,
dadurch **gekennzeichnet**, daß die Lasereinrichtung (1,10) einen zweiten Laserstrahl emittiert, dessen Wellenlänge kürzer als die des ersten Laserstrahls ist, und die zwischen etwa 170 nm und 550 nm liegt, und
daß der zweite Laserstrahl so auf das absorbierende Medium gerichtet ist daß das Medium teilweise ionisiert wird, wodurch die für die Herabsetzung der Durchbruchsschwelle nötigen Elektronen erzeugt werden, so daß die Auslösung des Laserinduzierten Durchbruchs durch den ersten Laserstrahl bei geringeren Laserpulsenergien erfolgt.

2. Vorrichtung nach Anspruch 1,
dadurch **gekennzeichnet**, daß das Medium der zu zertrümmernde feste Körper (7) ist.

3. Vorrichtung nach Anspruch 1,
dadurch **gekennzeichnet**, daß das Medium das den zu zertrümmernden festen Körper umgebende Fluid (4) ist.

4. Vorrichtung nach Anspruch 1,
dadurch **gekennzeichnet**, daß das Medium ein zusätzlich in den Bereich der Laserstrahlung eingebrachtes Element (61) ist.

5. Vorrichtung nach einem der Ansprüche 1 bis 4,
dadurch **gekennzeichnet**, daß beide Laserstrahlen gepulst werden.

6. Vorrichtung nach Anspruch 5,
dadurch **gekennzeichnet**, daß beide Laserstrahlen gleichzeitig und mit gleicher Frequenz gepulst werden.

7. Vorrichtung nach Anspruch 5,
dadurch **gekennzeichnet**, daß der Laserpuls des zweiten Laserstrahls vor dem Laserpuls des ersten Laserstrahls beginnt.

8. Vorrichtung nach einem der Ansprüche 1 bis 7,
dadurch **gekennzeichnet**, daß beide Laserstrahlen auf das absorbierende Medium fokussiert sind.

9. Vorrichtung nach einem der Ansprüche 3 bis 8,
dadurch **gekennzeichnet**, daß das Fluid Wasser, eine physiologische Kochsalzlösung, eine Zuckerlösung oder eine Suspension ist.

10. Vorrichtung nach Anspruch 9 in Verbindung mit Anspruch 8,
dadurch **gekennzeichnet**, daß der Brechungsindex des Fluids zur Einstellung des Fokuspunktes variiert wird.

11. Vorrichtung nach Anspruch 10,
dadurch **gekennzeichnet**, daß die Variation des Brechungsindex durch Änderung der Zusammensetzung, Konzentration und/oder Temperatur des Fluids erfolgt.

12. Vorrichtung nach einem der Ansprüche 1 bis 11,
dadurch **gekennzeichnet**, daß die Wellenlänge des ersten Laserstrahls zwischen 270 nm und 400 nm und die des zweiten Laserstrahls zwischen 700 nm und 1200 nm liegt.

13. Vorrichtung nach einem der Ansprüche 1 bis 12,
dadurch **gekennzeichnet**, daß der Lichtleiter Bestandteil eines an sich bekannten Endoskops ist.

14. Vorrichtung nach einem der Ansprüche 1 bis 13,
dadurch **gekennzeichnet**, daß ein einziger Lichtleiter das Licht mit einer Wellenlänge zwischen etwa 500 nm und etwa 1400 nm und das Licht mit einer Wellenlänge zwischen etwa 170 nm und 550 nm in das Organ bzw. Gefäß leitet.

15. Vorrichtung nach einem der Ansprüche 1 bis 14
dadurch **gekennzeichnet**, daß die Lasereinrichtung zwei Laser (1,10) aufweist, die Licht im Bereich zwischen etwa 500 nm und etwa 1400 nm und im Bereich zwischen etwa 170 nm und 550 nm erzeugen.

16. Vorrichtung nach einem der Ansprüche 1 bis 14,
dadurch **gekennzeichnet**, daß die Lasereinrichtung einen Laser aufweist, der Licht im Bereich zwischen etwa 500 nm und etwa 1400 nm erzeugt, und der zur Erzeugung von Licht im Bereich zwischen etwa 170 nm und 550 nm Frequenz-vervielfacht ist.

17. Vorrichtung nach einem der Ansprüche 1 bis 16,
dadurch **gekennzeichnet**, daß das Endoskop zumindest einen weiteren Kanal aufweist, durch den als Spüllösung ein Fluid wie Wasser, eine physiologische Kochsalzlösung, eine Zuckerlösung oder eine Suspension zuführbar ist.

18. Vorrichtung nach einem der Ansprüche 1 bis 17,
dadurch **gekennzeichnet**, daß das Endoskop in an sich bekannter Weise eine Einrichtung zur visuellen Beobachtung der zu zertrümmernden Konkremente aufweist.

19. Vorrichtung nach einem der Ansprüche 1 bis 18 oder nach dem Oberbegriff des Anspruchs 1,
dadurch **gekennzeichnet**, daß ein Drucksensor (9) vorgesehen ist, der die in dem Fluid erzeugte rücklaufende Stoßwelle erfaßt, und dessen Ausgangssignal eine Auswerteeinheit zur Erfassung des laserinduzierten Durchbruchs erfaßt.

20. Vorrichtung nach Anspruch 19,
dadurch **gekennzeichnet**, daß der Drucksensor extrakorporal angeordnet ist, und daß ein Druckübertragungsmedium vorgesehen ist.

21. Vorrichtung nach Anspruch 19 oder 20,
dadurch **gekennzeichnet**, daß zwei Drucksensoren vorgesehen sind.

## Claims

1. Device for breaking up a solid body (7) surrounded by a fluid (4), such concrements in the urinary system, in the gall-bladder or the biliary region or in the salivatory passage of living bodies, comprising
- a laser device (1, 10) which emits a first pulsed laser beam having a wavelength in the range from 500 nm approximately and 1400 nm approximately,
- a light guide (2) for said laser beam, and
- focusing means (21) joining said light guide for focusing said laser beam onto an absorbing medium such that, utilizing the photoacoustic effect or the optical breakdown in the absorbing medium or in the fluid surrounding the medium, respectively, shock waves are generated which break up the solid body
**characterized** in that said laser device (1, 10) emits a second laser beam whose wavelength is shorter than that of said first laser beam, which is in the range from 170 nm to 550 nm approximately, and
in that said second laser beam is directed towards the absorbing medium so as to partly ionize said medium, which produces the electrons necessary for a reduction of the breakdown threshold, such that the initiating of the laser-induced breakdown is effected by said first laser beam at reduced laser pulse energy levels.

2. Device according to Claim 1,
**characterized** in that the medium is the solid body (7) to be broken up.

3. Device according to Claim 1,
**characterized** in that the medium is the fluid (4) surrounding said solid body to be broken up.

4. Device according to Claim 1,
**characterized** in that said medium in an additional element (61) which is introduced into the laser radiation zone.

5. Device according to any of Claims 1 to 4,
**characterized** in that both laser beams are pulsed.

6. Device according to Claim 5,
**characterized** in that both laser beams are pulsed at the same time and at the same frequency.

7. Device according to Claim 5,
**characterized** in that the laser pulse of said second laser beam starts before the laser pulse of said first laser beam.

8. Device according to any of Claims 1 to 7,
**characterized** in that both laser beams are focused onto said absorbing medium.

9. Device according to any of Claims 3 to 8,
**characterized** in that said fluid is water, a physiological sodium chloride solution, a sugar solution, or a suspension.

10. Device according to Claim 9 in combination with Claim 8,
**characterized** in that the refractive index of said fluid is varied so as to adjust the focus.

11. Device according to Claim 10,
**characterized** in that the refractive index is varied by varying the composition, the concentration and/or the temperature of said fluid.

12. Device according to any of Claims 1 to 11,
**characterized** in that the wavelength of said first laser beam is in the range from 270 nm to 400 nm while the wavelength of said second laser beam is in the range from 700 nm to 1200 nm.

13. Device according to any of Claims 1 to 12,
**characterized** in that said light guide is an element of an endoscope known per se.

14. Device according to une any of Claims 1 to 13,
**characterized** in that a single light guide passes the light having a wavelength between 500 nm approximately and 1400 nm approximately, as well as the light having a wavelength between 170 nm approximately and 500 nm approximately into the organ or the vessel.

15. Device according to any of Claims 1 to 14,
**characterized** in that said laser device comprises two lasers (1, 10) for producing the light in the range from 500 nm approximately to 1400 nm approximately and in the range from 170 nm approximately to 550 nm approximately.

16. Device according to any of Claims 1 to 14,
**characterized** in that the laser device comprises a laser which produces light in the range from 500 nm approximately to 1400 nm approximately, and which is multiplied in terms of frequency for the production of light in the range from 170 nm to 500 nm approximately.

17. Device according to any of Claims 1 to 16,
**characterized** in that said endoscope comprises at least another passage through which a fluid such as water, a physiological sodium chloride solution, a sugar solution, or a suspension may be introduced as flushing solution.

18. Device according to any of Claims 1 to 17,
**characterized** in that said endoscope comprises, in a manner known per se, means for visual observation of concrements to be broken up.

19. Device according to any of Claims 1 to 18 or in accordance with the introductory clause of Claim 1,
**characterized** in that the pressure sensor (9) is provided for detecting the backward shock wave which is generated in said fluid, which furnishes an output signal for detection by an analyser for establishing the laser-induced breakdown.

20. Device according to Claim 19,
**characterized** in that said pressure sensor is disposed outside the body, and in that a pressure-transmitting medium is provided.

21. Device according to Claim 19 or 20,
**characterized** in that two pressure sensors are provided.

## Revendications

1. Dispositif pour détruire un corps solide (7) entouré d'un fluide (4), p.e. des concrétion dans le système urinaire, dans la région biliaire ou respectivement du canal cholédoque, ou dans le canal salivaire des corps vivants, comprenant
- un dispositif laser (1, 10) qui émet un premier rayon laser pulsé à une longueur d'onde dans la gamme entre 500 nm environ et 1400 nm environ,
- un guide de lumière (2) pour ledit rayon laser, et
- des moyens de focalisation (21) qui suivent ledit guide de lumière et focalisent le rayon laser sur un milieu absorbant de façon qu'en bénéficiant de l'effet photo-acoustique ou le claquage optique dans le milieu absorbant ou respectivement le fluide entourant ce milieu, des ondes de choc sont engendrées qui détruisent ledit corps solide,
**caractérisé** en ce que ledit dispositif laser (1, 10) émet un deuxième rayon laser dont la longueur d'onde est plus courte que celle dudit premier rayon laser et qui se trouve au dedans de la gamme entre 170 nm et 550 nm environ, et
en ce que ledit deuxième rayon laser est dirigé vers le milieu absorbant de façon que ledit milieu soit ionisé en partie afin d'engendrer les électrons requis pour un tel abaissement du seuil de claquage que le claquage induit par laser est déclenché par ledit premier rayon laser aux énergies d'impulsions laser réduites.

2. Dispositif selon la revendication 1,
**caractérisé** en ce que le milieu est ledit corps solide (7) à détruire.

3. Dispositif selon la revendication 1,
**caractérisé** en ce que le milieu est le fluide (4) entourant ledit corps solide à détruire.

4. Dispositif selon la revendication 1,
**caractérisé** en ce que ledit milieu est un élément (61) additionnel qui est introduit dans la zone du rayonnement laser.

5. Dispositif selon une quelconque des revendications 1 à 4,
**caractérisé** en ce que les deux rayons laser sont pulsés.

6. Dispositif selon la revendication 5,
**caractérisé** en ce que les deux rayons laser sont pulsés en même temps et à la même fréquence.

7. Dispositif selon la revendication 5,
**caractérisé** en ce que l'impulsion laser dudit deuxième rayon laser commence avant l'impulsion laser dudit premier rayon laser.

8. Dispositif selon une quelconque des revendications 1 à 7,
**caractérisé** en ce que les deux rayons laser sont focalisés sur ledit milieu absorbant.

9. Dispositif selon une quelconque des revendications 3 à 8,
**caractérisé** en ce que ledit fluide est de l'eau, de l'eau salé physiologique, de l'eau sucré, ou une suspension.

10. Dispositif selon la revendication 9 in combinaison avec la revendication 8,
**caractérisé** en ce que l'indice de réfraction dudit fluide est varié pour ajuster le foyer.

11. Dispositif selon la revendication 10,
**caractérisé** en ce que l'indice de réfraction est varié en variant la composition, la concentration et/ou la température dudit fluide.

12. Dispositif selon une quelconque des revendications 1 à 11,
**caractérisé** en ce que la longueur d'onde dudit premier rayon laser se trouve dans la gamme entre 270 nm et 400 nm pendant que la longueur d'onde dudit deuxième rayon laser est dans la gamme entre 700 nm et 1200 nm.

13. Dispositif selon une quelconque des revendications 1 à 12,
**caractérisé** en ce que ledit guide de lumière est un élément d'un endoscope connu en soi.

14. Dispositif selon une quelconque des revendications 1 à 13,
**caractérisé** en ce qu'un seul guide de lumière passe la lumière à une longueur d'onde entre 500 nm environ et 1400 nm environ, et la lumière à une longueur d'onde entre 170 nm environ et 500 nm environ dans l'organe ou vaisseau.

15. Dispositif selon une quelconque des revendications 1 à 14,
**caractérisé** en ce que le dispositif laser comprend deux lasers (1, 10) pour générer de la lumière dans la gamme entre 500 nm environ et 1400 nm environ et dans la gamme entre 170 nm environ et 550 nm environ.

16. Dispositif selon une quelconque des revendications 1 à 14,
**caractérisé** en ce que le dispositif laser comprend un laser qui engendre de la lumière dans la gamme entre 500 nm environ et 1400 nm environ, et qui est multiplié en fréquence pour la génération de lumière dans la gamme entre 170 nm et 500 nm environ.

17. Dispositif selon une quelconque des revendications 1 à 16,
**caractérisé** en ce que ledit endoscope comprend au moins un autre canal par lequel on peut introduire un fluide comme de l'eau, de l'eau salé physiologique, de l'eau sucré, ou une suspension comme solution de rinçage.

18. Dispositif selon une quelconque des Revendications 1 à 17,
**caractérisé** en ce que ledit endoscope comprend, de façon connue en soi, des moyens pour l'observation visuelle des concrétions à détruire.

19. Dispositif selon une quelconque des revendications 1 à 18 ou selon le préambule de la revendication 1,
**caractérisé** en ce qu'un capteur de pression (9) est prévu pour détecter l'onde de choc récurrente qui est générée dans ledit fluide, et dont le signal de sortie est détecté par un analyseur pour la détection du claquage induit par laser.

20. Dispositif selon la revendication 19,
**caractérisé** en ce que ledit capteur de pression est disposé à l'extérieur du corps, et en ce qu'un milieu de transfert de la pression est prévu.

21. Dispositif selon la revendication 19 ou 20,
**caractérisé** en ce que deux capteurs de pression sont prévus.
